# EUROPÄISCHE PATENTSCHRIFT

(11) **EP 1 438 937 B1**
(45) Veröffentlichungstag und Bekanntmachung des Hinweises auf die Patenterteilung: **08.03.2006**
(21) Anmeldenummer: 03023281.3
(22) Anmeldetag: 15.10.2003
(51) Int. Cl.: A61F 2/58, A61F 2/28, A61F 2/78

(54) **Fingerprothese**
Finger prosthesis
Prothèse de doigt

(30) Priorität: 17.01.2003 DE 10302432
(43) Veröffentlichungstag der Anmeldung: 21.07.2004
(73) Patentinhaber: ESKA Implants GmbH & Co., 23556 Lübeck (DE)
(72) Erfinder: Grundei, Hans, Dr.-Ing., 23558 Lübeck (DE)
(74) Vertreter: Fuchs Mehler Weiss & Fritzsche

(56) Entgegenhaltungen:
- WO-A-01/97718
- WO-A-02/13729
- DE-A- 19 650 816
- DE-A- 19 826 638
- DE-A- 19 854 762
- DE-C- 19 512 854
- DE-C- 19 931 882

## Beschreibung

Die vorliegende Erfindung betrifft eine Fingerprothese zu Adaption an einen Röhrenknochenstumpf eines Fingers, aufweisend ein in den Röhrenknochen implantierbares und dort fixierbares stielförmiges Implantat, mit welchem ein extrakorporaler Ersatzkörper koppelbar ist. Die Finger prothese weist also eine sogenannte Endo/Exo-Prothese auf, die im Röhrenknochen verankert ist und durch die Haut und Bindegewebe nach außen hin durchtritt. Ein derartiges Implantat ist bekannt aus der DE 198 26 638 C2. Der darin beschriebene Adapter findet in erster Linie Einsatz bei einer Beinprothese wie sie aus der DE 198 45 191 C1 bekannt ist.

Krankheits- oder unfallbedingt kommt es immer wieder zu Verlusten eines ganzen Fingers oder von Teilen eines Fingers. Der Verlust eines Daumens oder von Teilen eines Daumens haben besonders gravierende Folgen aufgrund der Gegenüberstellung des Daumens gegen die anderen Finger, der so genannten Oppositionsstellung. Diese Oppositionsstellung erlaubt erst ein Greifen mit der Hand und stellt die wichtigste Werkzeugfunktion der Hand dar. Eine sogenannte transkutane Prothese, also eine Endo/Exoprothese ist ebenfalls bekannt aus der WO 01/97718 A1. Diese soll auch geeignet sein als Fingerprothese zu dienen. Konkrete Hinweise auf die Konstruktion hierfür sind dieser Druckschrift jedoch nicht zu entnehmen.

Die Erfindung hat es sich nun zur Aufgabe gestellt, hier Abhilfe zu schaffen, also eine konkrete Fingerersatzprothese anzugeben, die es gestattet, die Funktionstüchtigkeit eines teilamputierten Fingers, insbesondere des Daumens, weitgehend wiederherzustellen.

Gelöst wird diese Aufgabe durch eine Fingerprothese mit den Merkmalen des Anspruchs 1. Weitere vorteilhafte Weiterbildungen ergeben sich aus den Unteransprüchen.

Erfindungsgemäß weist die Fingerersatzprothese die folgenden Merkmale auf:
- Das stielförmige Implantat ist hohl und eine in seinem Ansatz als konische Klemmhülse ausgebildete Durchbohrung im Kopf bis hin zum hohlen Stiel sowie eine Gewindebohrung im unteren Ende des Stieles ist vorgesehen,
- eine Gewindehülse ist vorgesehen, welche an ihrem Fußende ein Gewinde aufweist, welche mit der Gewindebohrung im unteren Ende des Stieles verschraubbar ist, die eine solche Länge, die mindestens der Länge des das Einwachsen von Knochentrapekeln ermöglichende Oberflächenbereiches entspricht, und eine Innengewindebohrung im Kopfende aufweist, und
- es ist ein Adapterstück vorgesehen, das in die konische Klemmverbindung bringbar ist mit einem sich außerhalb des stielförmigen Implantates erweiternden Kopf, bei dem durch sein Inneres eine Gewindeschraube setzbar ist, die mit der Innengewindebohrung im Kopfende der Gewindehülse verschraubbar ist.

Besonders bevorzugt kommt auch bei dieser Fingerprothese das schon erwähnte feinmaschige Gewebe zum Einsatz, um eine Keimschranke durch Eingranulieren von Bindegewebe und Restmuskulatur zwischen Knochenhaut und Oberhaut zu erzeugen. Der sich erweiternde Kopf des Adapterstückes sorgt für eine Kompression der Oberhaut bei Herstellung der Verbindung zwischen dem Adapterstück und dem stielförmigen Implantat und spielt somit eine wichtige Rolle hinsichtlich der Abdichtung des Übergangs von extra- nach intrakorporal. Die Gewindehülse dient nicht nur zur Verschraubung des Adapterstückes mit dem stielförmigen Implantat, sondern fungiert auch als Platzhalter für ein Extraktionsgewinde für den Fall, dass das Implantat entfernt werden muss. In die Gewindebohrung im unteren Ende des Stieles des Implantates würde dann ein Gewinde eines Extraktionswerkzeuges geschraubt werden und das Implantat aus dem Röhrenknochen(stumpf) herausgezogen werden.

Eine besonders bevorzugte Ausführungsform besteht darin, dass das stielförmige Implantat zumindest teilweise mit einer offenmaschigen dreidimensionalen Raumnetzstruktur bedeckt ist.

Alternativ hierzu ist das stielförmige Implantat aus einem zu einem Rohr geformten offenen maschenförmigen Netzwerk gebildet. Diese und die vorerwähnte Ausführungsform gestattet nicht nur ein Einwachsen von Knochentrabekeln, sondern ein regelrechtes Durchwachsen der Oberfläche des Implantates mit den Knochentrabekeln, wodurch für einen besonders festen Halt des Implantates im Röhrenknochen(stumpf) gesorgt wird.

Der Problematik, dass es sich bei der Prothese um eine transkutane Prothese handelt und daher grundsätzlich die Gefahr einer Verkeimung besteht, wird vorteilhaft durch folgende Maßnahme begegnet:

Im Ankoppelungsbereich des stielförmigen Implantates mit der extrakorporalen Struktur ist ein feinmaschiges Gewebe über das stielförmige Implantat gestülpt. Dies hat nun die folgende Bewandtnis:

Während des Einheilprozesses verwächst knochenseitig Restmuskelgewebe in das feinmaschige Gewebe, während von der anderen Seite des feinmaschigen Gewebes Bindegewebe eingranuliert, also einwächst. Das feinmaschige Gewebe bildet also ein Sperrgerüst aus Bindegewebe und Restmuskulatur, das sich zwischen der Knochenhaut und der Oberhaut befindet, so dass auf diese Weise eine Keimsperre geschaffen wird.

Das feinmaschige Gewebe besteht vorzugsweise aus einer metallischen Wolle, besonders bevorzugt aus Titan.

Die Koppelung zwischen dem stielförmigen Implantat und dem extrakorporalen Ersatzkörper erfolgt besonders bevorzugt über eine konische Klemmverbindung. Hierzu muss die extrakorporale Struktur durch die Oberhaut hindurch mit dem stielförmigen Implantat in die konische Klemmverbindung gebracht werden. Hierzu kann das Implantat beispielsweise eine konische Klemmhülse und der extrakorporale Ersatzkörper einen entsprechenden konischen Klemmzapfen aufweisen. Auch die umgekehrte Ausbildung ist möglich.

Der extrakorporale Ersatzkörper ist vorzugsweise als Restfinger ausgebildet, so dass die Funktionen der amputierten Fingerteile bestmöglich ausgeübt werden können.

Die Erfindung wird anhand eines konkreten Ausführungsbeispiels gemäß den Zeichnungen näher erläutert. Hierbei zeigt:
- Fig. 1: das Skelett einer menschlichen Hand mit implantiertem stielförmigen Implantat im Os metacarpale I und angedeuteten amputierten Fingerteilen,

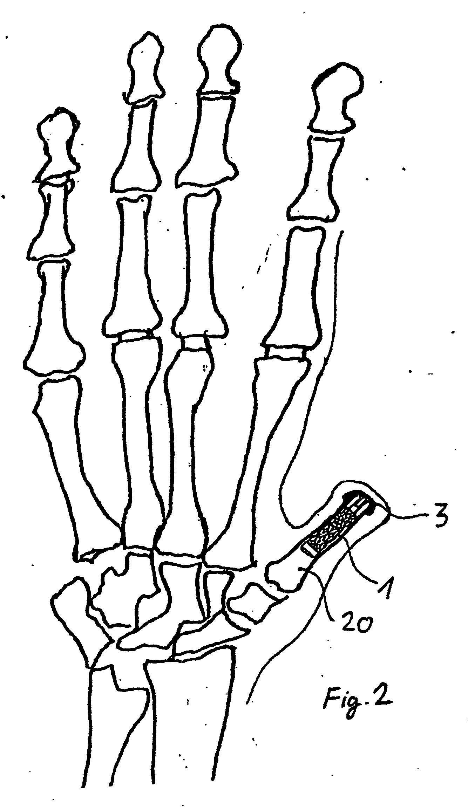

- Fig. 2: eine ähnliche Ansicht ohne amputierte Fingerteile,
- Fig. 3: eine vergrößerte Ansicht des stielförmigen Implantates im Os metacarpale I mit angekoppelter Restfingerersatzstück,
- Fig.4: eine besonders bevorzugte Ausführungsform der Fingerprothese,
- Fig. 5: eine Aufsicht auf das feinmaschige Gewebe, und
- Fig. 6: eine ähnliche Ansicht wie die Figuren 1 bis 3 der ganzen Hand mit der komplettierten Fingerprothese.

Nachfolgend bezeichnen gleiche Bezugszeichen gleiche Teile.

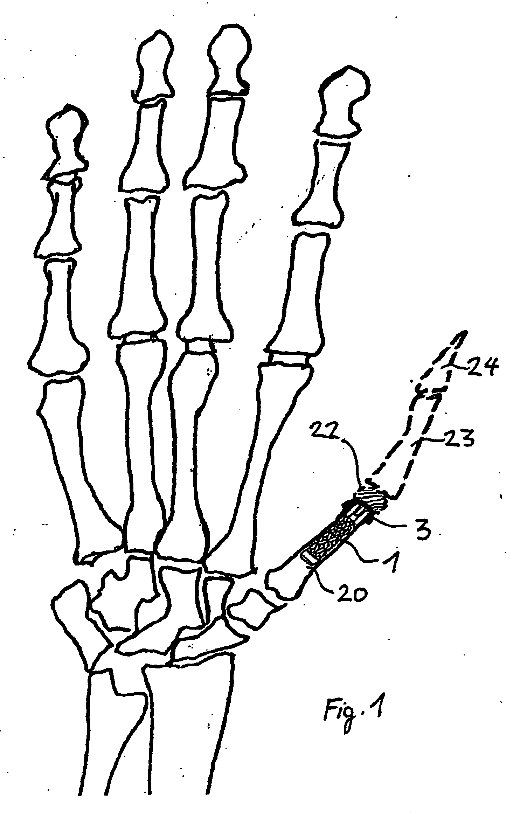

Einen ersten Überblick verschafft Fig. 1. Darin ist das Skelett einer menschlichen Hand dargestellt. Im vorliegenden Fall sind der Phalanx prox. pollicis und Phalanx dist. pollicis gestrichelt dargestellt, womit angedeutet werden soll, dass diese abgetrennt worden sind, zusammen mit dem Grundgelenk 22 des Daumens.

In Os metacarpale I 20 ist nun das erfindungsgemäße stielförmige Implantat 1 eingesetzt. Über das proximale Ende des Implantates 1 ist ein feinmaschiges Gewebe 3 gestülpt, in welches von unten zwischen der Knochenhaut und der Oberhaut Bindegewebe und Restmuskulatur eingranuliert, um eine Keimsperre zu bilden.

Fig._{.}2 zeigt diesen Sachverhalt nochmals ohne die abgetrennten Daumenteile.

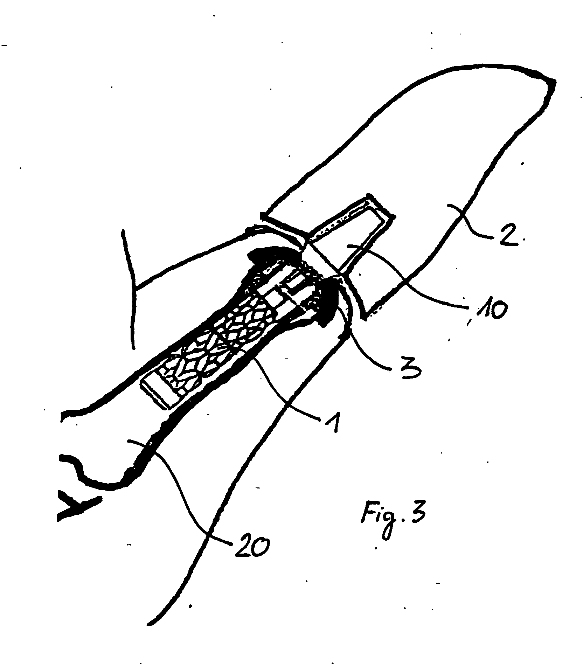

In Fig. 3 ist schematisch die gesamte Fingerprothese dargestellt. Im Ankoppelungsbereich, also dort, wo das feinmaschige Gewebe 3 über das stielförmige Implantat 1 gestülpt ist, durchdringt das Adapterstück 10 die Oberhaut des Daumenstumpfes und greift in eine konische Klemmhülse, die im Kopf des Implantates 1 ausgebildet ist.

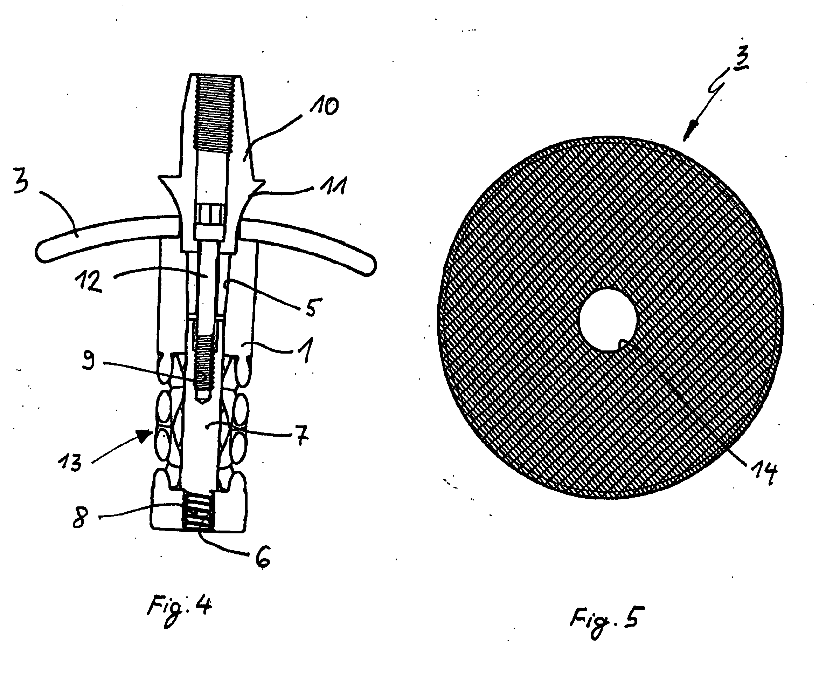

Das Adapterstück 10 trägt den exkorporale Ersatzkörper in Form des Restdaumens. Einzelheiten des Implantates ergeben sich nun aus Fig. 4.

Kern der Fingerprothese ist zunächst das stielförmige Implantat 1, das vorliegend aus einem hohlen metallischen Stiel besteht. Dieser besteht im Wesentlichen bereichsweise aus einem offenen maschenförmigen Netzwerk 13. Das stielförmige Implantat 1 weist in seinem Kopf eine Durchbohrung 5 bis hin zum hohlen Stiel sowie eine Gewindebohrung 6 im unteren Ende des Stieles auf. In letztere Gewindebohrung kann ein Extraktionsgewinde (nicht dargestellt) eines Extraktionswerkzeuge geschraubt werden, wenn das Implantat entfernt werden muss. Vorliegend aber ist gewissermaßen als Platzhalter hierfür ein Gewinde 8 geschraubt, welches zu der Gewindehülse 7 gehört. Die Gewindehülse 7 weist eine glatte Oberfläche mit einer Länge auf, die wenigstens so groß ist, wie die Länge des Bereichs des maschenförmigen Netzwerkes 13. In ihrem Kopfende weist die Gewindehülse 7 eine Innengewindebohrung 9 auf. Mit dieser verschraubt ist eine Gewindeschraube 12, die durch das Innere des Adapterstückes 10 geführt ist. Auf diese Weise kann das Adapterstück 10 fest mit dem stielförmigen Implantat 1 verbunden werden. Zusätzlich ist eine konische Klemmverbindung zwischen beiden Teilen vorgesehen. Hierzu ist die Gewindebohrung 5 als konische Klemmhülse ausgebildet, während das Ende des Adapterstückes 10 als konischer Klemmzapfen ausgebildet ist.

Der Kopf 11 des Adapterstückes 10 erweitert sich außerhalb des stielförmigen Implantates 1. Der Grund hierfür wird weiter unten näher er läutert.

Über das stielförmige Implantat 1 ist ein feinmaschiges Gewebe 3 gestülpt. In dieses granuliert von unten Bindegewebe und von oben Restmuskulatur ein. Auf diese Weise wird eine Keimschranke erzeugt. Zwischen der Oberseite des feinmaschigen Gewebes 3 und dem sich erweiternden Kopf 11 des Adapterstückes 10 kommt nach der Implantation Oberhaut zu liegen. Aufgrund der speziellen Ausbildung des sich erweiternden Kopfes 11 wird die Oberhaut an dieser Stelle bei Herstellung der Verbindung zwischen dem Adapterstück 10 und dem stielförmigen Implantat 1 komprimiert, wodurch die Dichtigkeit des Über gangs von intrakorporal nach extrakorporal deutlich erhöht wird.

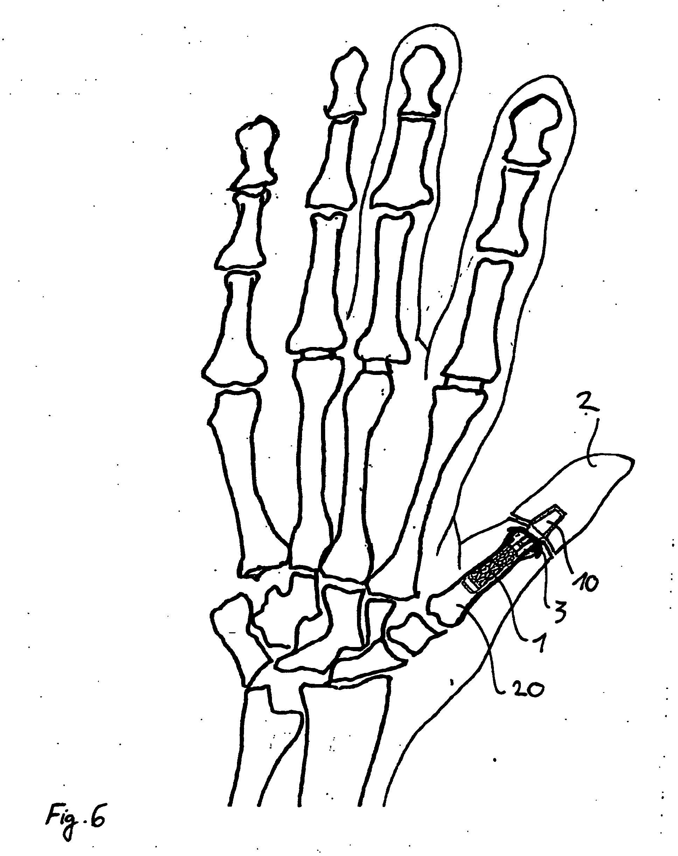

Zur Abrundung ist in Fig. 5 das feinmaschige Gewebe 3 vergrößert dargestellt. Dieses besteht im Wesentlichen aus einer Scheibe, weiche zentral eine Ausnehmung 14 aufweist. Durch diese Ausnehmung 14 wird bei dem Zusammenstellen der Fingerprothese der Kopf 11 des Adapterstückes 10 gesetzt.

Zur Abrundung zeigt Fig. 6 nochmals ein Handskelett mit der vollständigen Fingerprothese.

## Patentansprüche

1. Fingerprothese zur Adaption an einen Röhrenknochenstumpf (20) eines Fingers, aufweisend ein in den Röhrenknochen (21) implantierbares und dort fixierbares stielförmiges Implantat (1), mit welchem ein extrakorporaler Ersatzkörper (2) koppelbar ist,
**dadurch gekennzeichnet,**
- **dass** das stielförmige Implantat (1) hohl ist und eine in seinem Ansatz als konische Klemmhülse ausgebildete Durchbohrung (5) im Kopf bis hin zum hohlen Stiel sowie eine Gewindebohrung (6) im unteren Ende des Stieles vorgesehen ist,
- **dass** eine Gewindehülse (7) vorgesehen ist, welche an ihrem Fußende ein Gewinde (8) aufweist, welche mit der Gewindebohrung (6) im unteren Ende des Stieles verschraubbar ist, mit einer Länge, die mindestens der Länge des das Einwachsen von Knochentrapekeln ermöglichende Oberflächenbereiches entspricht, und mit einer Innengewindebohrung (9) im Kopfende, und
- **dass** ein Adapterstück (10) vorgesehen ist, das in die konische Klemmverbindung bringbar ist mit einem sich außerhalb des stielförmigen Implantates (1) erweiternden Kopf (11), bei dem durch sein Inneres eine Gewindeschraube (12) setzbar ist, die mit der Innengwindebohrung (9) im Kopfende der Gewindehülse (7) verschraubbar ist.

2. Fingerprothese nach Anspruch 1, bei der das stielförmige Implantat (1) zumindest teilweise eine solche Oberfläche aufweist, die ein Einwachsen von Knochentrapekeln ermöglicht.

3. Fingerprothese nach Anspruch 2, bei der das stielförmige Implantat (1) mit einer offenmaschigen, dreidimensionalen Raumnetzstruktur bedeckt ist.

4. Fingerprothese nach Anspruch 2, bei der das stielförmige Implantat (1) aus einem zu einem Rohr geformten offenen maschenförmigen Netzwerk gebildet ist.

5. Fingerprothese nach einem der Ansprüche 1 bis 4, bei der im Ankopplungsbereich des stielförmigen Implantates (1) mit dem extrakorporalen Ersatzkörper (2) ein feinmaschiges Gewebe (3) über das stielförmige Implantat gestülpt ist.

6. Fingerprothese nach Anspruch 5, bei der das Gewebe aus einer metallischen Wolle besteht.

7. Fingerprothese nach Anspruch 6, bei der die metallische Wolle aus Titan besteht.

8. Fingerprothese nach einem der Ansprüche 1 bis 7, bei der die Kopplung zwischen dem stielförmigen Implantat und dem extrakorporalen Ersatzkörper über eine konische Klemmverbindung erfolgt.

9. Fingerprothese nach einem der Ansprüche 1 bis 8, bei der der extrakorporale Ersatzkörper (2), als Restfinger ausgebildet ist.

## Claims

1. Finger prosthesis for adaptation to a tubular bone stump (20) of a finger, having a stem-like implant (1) which can be implanted in the tubular bone (21) and can be fixed there, and with which an extracorporeal replacement body (2) can be coupled, **characterised in that**
- the stem-like implant (1) is hollow and a bore (5) designed as a conical clamping sleeve in its attachment is provided in the head as far as the hollow stem and a threaded bore (6) is provided in the lower end of the stem,
- **in that** a threaded sleeve (7) is provided, which has at its base end a thread (8) which can be screwed with the threaded bore (6) in the lower end of the stem, with a length which corresponds at least to the length of the surface region facilitating the inward growth of bone trabeculae, and with an inner threaded bore (9) in the head end, and
- **in that** an adapter piece (10) is provided which can be introduced into the conical clamping connection with a head (11) expanding outside of the stem-like implant (I), in which head (11) a threaded screw (12) can be placed through its interior and which can be screwed with the inner threaded bore (9) in the head end of the threaded sleeve (7).

2. Finger prosthesis according to claim 1, in which the stem-like implant (1) has at least partly such a surface which facilitates inward growth of bone trabeculae.

3. Finger prosthesis according to claim 2, in which the stem-like implant (1) is covered by an open-mesh, three-dimensional spatial network structure.

4. Finger prosthesis according to claim 2, in which the stem-like implant (1) is formed from an open mesh-like network shaped to form a tube.

5. Finger prosthesis according to one of claims 1 to 4, in which a fine-mesh fabric (3) is inverted over the stem-like implant in the coupling region of the stem-like implant (1) with the extracorporeal replacement body (2).

6. Finger prosthesis according to claim 5, in which the fabric consists of a metallic wool.

7. Finger prosthesis according to claim 6, in which the metallic wool consists of titanium.

8. Finger prosthesis according to one of claims 1 to 7, in which the coupling between the stem-like implant and the extracorporeal replacement body is effected via a conical clamping connection.

9. Finger prosthesis according to one of claims I to 8, in which the extracorporeal replacement body (2) is designed as the remainder of a finger.

## Revendications

1. Prothèse de doigt destinée à être adaptée sur un moignon d'os long (20) d'un doigt, présentant un implant en forme de tige (1) que l'on peut implanter et fixer dans l'os long (21) et auquel on peut coupler un corps de substitution extracorporel (2),
**caractérisée en ce que**
- l'implant en forme de tige (1) est creux et est muni d'un perçage (5) présentant, à son extrémité, la forme d'une douille de serrage conique dans la tête jusqu'à la tige creuse et d'un perçage taraudé (6) à l'extrémité inférieure de la tige,
- il est prévu une douille taraudée (7) qui présente, à son extrémité de pied, un filet (8), qui peut être vissé dans le perçage taraudé (6) à l'extrémité inférieure de la tige et qui a une longueur qui correspond au moins à la longueur de la zone de surface permettant la croissance de travées osseuses, et un perçage taraudé (9) à l'extrémité de tête, et
- il est prévu une pièce adaptatrice (10) que l'on peut placer dans la liaison de serrage conique avec une tête s'élargissant (11) en dehors de l'implant en forme de tige (1) et à l'intérieur de laquelle on peut placer une vis (12) que l'on peut visser dans le perçage taraudé (9) de l'extrémité de tête de la douille taraudée (7).

2. Prothèse de doigt selon la revendication 1, dans laquelle l'implant en forme de tige (1) présente au moins partiellement une surface qui permet une croissance de travées osseuses.

3. Prothèse de doigt selon la revendication 2, dans laquelle l'implant en forme de tige (1) est recouvert d'une structure réticulée tridimensionnelle à mailles ouvertes.

4. Prothèse de doigt selon la revendication 2, dans laquelle l'implant en forme de tige (1) est constitué d'un réseau à mailles ouvertes conformé en tube.

5. Prothèse de doigt selon l'une quelconque des revendications 1 à 4, dans laquelle un tissu à fines mailles (3) est appliqué sur l'implant en forme de tige dans la zone de couplage de l'implant en forme de tige (1) avec le corps de substitution extracorporel (2).

6. Prothèse de doigt selon la revendication 5, dans laquelle le tissu est constitué d'une laine métallique.

7. Prothèse de doigt selon la revendication 6, dans laquelle la laine métallique est constituée de titane.

8. Prothèse de doigt selon l'une quelconque des revendications 1 à 7, dans laquelle le couplage entre l'implant en forme de tige et le corps de substitution extracorporel s'effectue via une liaison de serrage conique.

9. Prothèse de doigt selon l'une quelconque des revendications 1 à 8, dans laquelle le corps de substitution extracorporel (2) se présente sous la forme d'un doigt partiel.
